(19) **Europäisches Patentamt European Patent Office Office européen des brevets**

(11) **EP 4 006 009 B1**

(12) **EUROPEAN PATENT SPECIFICATION**

(45) Date of publication and mention
of the grant of the patent:
**29.07.2026 Bulletin 2026/31**

(51) International Patent Classification (IPC):
**C07C 253/30** (2006.01)    **C07C 253/34** (2006.01)
**C07C 255/04** (2006.01)    **C07C 255/09** (2006.01)
**B01J 31/02** (2006.01)

(21) Application number: **21851944.5**

(22) Date of filing: **09.06.2021**

(52) Cooperative Patent Classification (CPC):
(C-Sets available)
**C07C 253/30; B01J 31/0262; B01J 31/0267;
C07C 253/34;** Y02P 20/52                (Cont.)

(86) International application number:
**PCT/KR2021/007222**

(87) International publication number:
**WO 2022/080621 (21.04.2022 Gazette 2022/16)**

(54) **METHOD FOR PREPARING ACRYLONITRILE DIMER**

VERFAHREN ZUR HERSTELLUNG VON ACRYLNITRIL-DIMER

PROCÉDÉ DE PRÉPARATION D'UN DIMÈRE D'ACRYLONITRILE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.10.2020 KR 20200131026**

(43) Date of publication of application:
**01.06.2022 Bulletin 2022/22**

(73) Proprietor: **LG CHEM, LTD.
Yeongdeungpo-gu,
Seoul 07336 (KR)**

(72) Inventors:
• **AN, Yujin**
  **Daejeon 34122 (KR)**
• **KIM, Wonseok**
  **Daejeon 34122 (KR)**
• **JUNG, Hyunchul**
  **Daejeon 34122 (KR)**
• **PARK, Sae Hume**
  **Daejeon 34122 (KR)**
• **OH, Wan Kyu**
  **Daejeon 34122 (KR)**

(74) Representative: **Goddar, Heinz J.
Boehmert & Boehmert
Anwaltspartnerschaft mbB
Pettenkoferstrasse 22
80336 München (DE)**

(56) References cited:
**WO-A1-2013/095853    WO-A1-93/10082
WO-A1-93/10082       US-A- 3 950 370
US-A- 4 316 857       US-A- 4 639 539
US-A- 4 639 539       US-A- 4 958 042
US-A- 5 332 844**

(52) Cooperative Patent Classification (CPC): (Cont.)

C-Sets
**C07C 253/30, C07C 255/04;**
**C07C 253/30, C07C 255/09;**
**C07C 253/34, C07C 255/04;**
**C07C 253/34, C07C 255/09**

**Description**

**[Technical Field]**

The present disclosure relates to a method for preparing acrylonitrile dimer.

**[Background Art]**

Acrylonitrile dimer such as 2-methyleneglutaronitrile (MGN), 1,4-dicyanobutene(DCB) and/or adiponitrile (AND) is a precursor material used in the preparation of a main monomer of nylon 66, a rust inhibitor and a curing accelerator for rubber materials, and besides this, it is usefully used in various other fields. As a method of obtaining a dimer of acrylonitrile, a method of dimerizing acrylonitrile in the presence of a phosphorus-based catalyst is commonly used.

However, while the phosphorus-based catalyst used for the reaction has excellent reactivity and selectivity, the reaction proceeds under a homogeneous catalyst system, which would be difficult to separate the catalyst. Distillation is used as a general catalyst recovery method, but due to the high temperature and pressure during distillation, it is likely to cause a decomposition of the catalyst or a side reaction of dimer which is a product.

Thus, in order to effectively extract the catalyst from a homogeneous catalyst system without catalyst decomposition and side reactions, liquid-liquid extraction (LLE) may be used. As an example, U.S. Patent No. 4,639,539 provides a process of extracting a product with formamide and a catalyst with toluene by using formamide as an extraction solvent and subjecting to phase separation of formamide and toluene which is a reaction solvent. The phase equilibrium system of formamide and toluene has a partition coefficient that is not bad for catalyst separation, but there is a disadvantage in that the product is uniformly dissolved in both of the two solvents and thus, a separation process in each solvent is additionally required to recover the product.

[Prior Art Literature]

[Patent Literature]
(Patent Literature 1) U.S. Patent No. 4,639,539
WO 93/10082 A1 discloses dimerizing acrylonitrile in the presence of a phosphorus-based catalyst, an inert proton-donating solvent and a non-hydroxylic solvent, wherein the dimerization products and an additional solvent are mixed, and sent to a means that separates the mixture into a first phase comprising the solvents, catalyst and acrylonitrile and a second phase comprising the dimer and polymeric byproducts.

US 4,639,539 A discloses the production of 1,4-dicyano-1-butene from acrylonitrile where the acrylonitrile is catalytically dimerized in the presence of a phosphinite or a phosphonite, an inert hydrocarbon reaction solvent and a non-interfering proton-donating solvent; and the dimerized product is thereafter separated from the catalyst by extraction, wherein an amide is used.

WO 2013/095853 A1 discloses a process for recovering diphosphite-containing compounds from a feed mixture, comprising diphosphite-containing compounds, organic mononitriles, organic dinitriles and a Lewis acid in a multistage countercurrent liquid-liquid extractor with extraction solvent comprising aliphatic hydrocarbon, cycloaliphatic hydrocarbon or a mixture thereof.

**[Disclosure]**

**[Technical Problem]**

In order to solve the above-mentioned problems, the present disclosure provides a method for preparing acrylonitrile dimer which utilizes liquid-liquid extraction(LLE) to block the possibility of a decomposition of the catalyst and a side reaction of the product and uses a solvent that is effective for extraction of only the catalyst.

**[Technical Solution]**

The present disclosure provides a method for preparing acrylonitrile dimer described below:
A method for preparing acrylonitrile dimer comprising the steps of:

reacting acrylonitrile in the presence of a reaction solvent, a proton-donating solvent, and a phosphorus-based catalyst to prepare an acrylonitrile dimer (step 1);
removing the reaction solvent, the proton-donating solvent and unreacted acrylonitrile (step 2A); and
adding an amine-based solvent to a reaction mixture containing the acrylonitrile dimer prepared in step 1 and extracting the same to separate the acrylonitrile dimer and the phosphorus-based catalyst (step 2),
wherein the reaction solvent is at least one selected from the group consisting of toluene, cyclohexane, 1,2-dichloroethane, 1,4-dioxane, chlorobenzene, and xylene,
the proton-donating solvent is at least one selected from the group consisting of isopropyl alcohol (IPA), butanol, benzyl alcohol, and cyclohexanol,
the phosphorus-based catalyst is an alkyl diphenyl phosphinite having 1 to 10 carbon atoms, and
the amine-based solvent is at least one selected from the group consisting of triethylamine and trioctylamine.

Further embodiments are disclosed in the dependent claims.

## [Advantageous Effects]

According to the present disclosure, a possibility that a catalyst is decomposed or side reactions proceed in the process of recovering the catalyst can be remarkably reduced, and the catalyst of a single catalyst system can be effectively separated, thereby preparing an acrylonitrile dimer.

## [Mode for Invention]

The present disclosure provides the following preparation method in order to efficiently extract a phosphorus-based catalyst used in the preparation of acrylonitrile dimer:
A method for preparing acrylonitrile dimer comprising the steps of:

reacting acrylonitrile in the presence of a reaction solvent, a proton-donating solvent, and a phosphorus-based catalyst to prepare an acrylonitrile dimer (step 1);
removing the reaction solvent, the proton-donating solvent and unreacted acrylonitrile (step 2A); and
adding an amine-based solvent to a reaction mixture containing the acrylonitrile dimer prepared in step 1 and extracting the same to separate the acrylonitrile dimer and the phosphorus-based catalyst (step 2),
wherein the reaction solvent is at least one selected from the group consisting of toluene, cyclohexane, 1,2-dichloroethane, 1,4-dioxane, chlorobenzene, and xylene,
the proton-donating solvent is at least one selected from the group consisting of isopropyl alcohol (IPA), butanol, benzyl alcohol, and cyclohexanol,
the phosphorus-based catalyst is an alkyl diphenyl phosphinite having 1 to 10 carbon atoms, and
the amine-based solvent is at least one selected from the group consisting of triethylamine and trioctylamine.

Hereinafter, the present disclosure will be described in detail for each step.

## (Step 1: step of preparing acrylonitrile dimer)

Step 1 of the present disclosure is a step of preparing an acrylonitrile dimer, which is a step of reacting acrylonitrile in the presence of a reaction solvent, a proton-donating solvent, and a phosphorus-based catalyst. Step 1 is not particularly limited as long as it is a conventional method for preparing an acrylonitrile dimer in solution in the presence of a phosphorus-based catalyst. Here, the acrylonitrile dimer includes 2-methyleneglutaronitrile(MGN), 1,4-dicyanobutene (DCB) and/or adiponitrile (AND), and the like.
The reaction solvent is at least one selected from the group consisting of toluene, cyclohexane, 1,2-dichloroethane, 1,4-dioxane, chlorobenzene, and xylene. These organic solvents inactive to the reaction may be used either singly or as a mixture of two or more solvents. More preferably, the solvent may be toluene.
The proton-donating solvent is a material that serves to donate protons in the preparation reaction of acrylonitrile dimer, and is at least one selected from the group consisting of isopropyl alcohol (IPA), butanol, benzyl alcohol, and cyclohexanol, and more preferably, it may be isopropanol (IPA).
The phosphorus-based catalyst is an alkyl diphenyl phosphinite having 1 to 10 carbon atoms. More preferably, the phosphorus-based catalyst may be isopropyl diphenylphosphinite, or ethyl diphenylphosphinite. Here, the alkyl group may be a straight-chain or a branched, and the carbon number is 1 to 10. Specific examples include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethyl-propyl, isohexyl, 2-methylpentyl, 4-methylhexyl, 5-methylhexyl, and the like, without being limited thereto.

**(Step 2: step of separating acrylonitrile dimer and phosphorus-based catalyst)**

Step 2 of the present disclosure is a step of separating the acrylonitrile dimer prepared in step 1 and the phosphorus-based catalyst remaining after preparation by liquid-liquid extraction (LLE), and in particular, an amine solvent is used as the extraction solvent in order to efficiently extract only the phosphorus-based catalyst from the reaction mixture in which the acrylonitrile dimer, which is a reaction product, and the phosphorus-based catalyst are mixed. The "reaction mixture" is a mixture in which the step of preparing the acrylonitrile dimer of step 1 has been performed, and may include a reaction solvent, a proton-donating solvent, a phosphorus-based catalyst, an acrylonitrile dimer which is a reaction product, and acrylonitrile which is an unreacted material.

The extraction solvent is a solvent used for extracting a target material in liquid-liquid extraction (LLE). The liquid-liquid extraction method is a method of extracting a target material mixed in a liquid sample by a distribution difference using a solvent (extraction solvent) having a higher affinity with the target material than the solvent of a sample. At this time, the liquid sample containing the target material and the extraction solvent must not be mixed with each other, and the extraction solvent is preferably a highly volatile solvent that can be easily removed after extraction. Therefore, in step 2, an amine-based solvent is used as an extraction solvent.

In the amine-based solvent, 2-methyleneglutaronitrile (MGN), 1,4-dicyanobutene (DCB) and/or adiponitrile(AND), and the like, which are the products of step 1, are not well mixed, and thus are preferable as an extraction solvent. In addition, the partition coefficient of the product of step 1 is higher compared to that of the alkane-based solvent used for extraction of the phosphorus-based catalyst in the general process for preparing acrylonitrile dimer, so that the extraction efficiency is improved, thereby increasing the recovery rate of the phosphorus-based catalyst.

In one example, the partition coefficient ($K_{AM/ADN}$) of the amine-based solvent-adiponitrile of the phosphorus-based catalyst represented by Equation 1 below at 50°C and 101.3 kPa (760 torr) may be 2.5 or more, 2.6 or more, 2.7 or more, or 2.8 or more. The upper limit of the partition coefficient is not particularly limited, but may be, for example, 6.0 or less, 5.0 or less, or 4.0 or less.

$$[\text{Equation 1}]$$

$$K_{AM/ADN} = C_{AM}/C_{ADN}$$

in Equation 1,

> $C_{AM}$ is the mass fraction of the phosphorus-based catalyst in the amine-based solvent, and
> $C_{ADN}$ is the mass fraction of phosphorus-based catalyst in adiponitrile.

Further, the amine-based solvent should have low miscibility with the acrylonitrile dimer so that only the phosphorus-based catalyst can be selectively extracted.

The amine-based solvent may preferably have a density of 0.950 g/cm$^3$ or less, or 0.930 g/cm$^3$ or less. Since most of the dimers produced by the dimerization reaction of acrylonitrile are 1,4-dicyanobutene having a density of 1.0 g/cm$^3$ level, acrylonitrile dimer and phase separation may occur smoothly in the extraction step when the $C_{4-10}$ unsaturated hydrocarbon satisfies the above density range.

The amine-based solvent is at least one selected from the group consisting of triethylamine and trioctylamine. Most preferably, the amine-based solvent may be triethylamine.

In step 2, the amount of the amine-based solvent used may be adjusted according to the amount of the reaction mixture to be extracted. In one example, the amine-based solvent may be used in an amount of 80 to 150 parts by weight, or 90 to 130 parts by weight, or 100 to 120 parts by weight, based on 100 parts by weight of the reaction mixture obtained in step 1.

When the amount of the amine-based solvent used is too small, the phosphorus-based catalyst may not be sufficiently extracted and thus, the catalyst component may remain on the acrylonitrile dimer. Conversely, when the amine-based solvent is excessively used, the acrylonitrile dimer may be extracted together, or phase separation from the acrylonitrile dimer may be difficult, and so it is preferable to satisfy the above range.

On the other hand, preferably, step 2 may be performed at 30°C or more and 80°C or less. If the temperature for performing step 2 is less than 30°C, there is a problem that the extraction efficiency of the target material is lowered, and when the temperature exceeds 80°C, there is a problem that the extraction solvent is volatilized.

Preferably, step 2 may be performed for 30 minutes or less. When the extraction is performed for 30 minutes or more, there is a possibility that the process efficiency will decrease. Further, step 2 may be performed for 1 minute or more, 2 minutes or more, or 3 minutes or more.

When the time for performing step 2 is less than 1 minute, the phosphorus-based catalyst may not be sufficiently extracted with the catalyst solvent.

Therefore, the reaction amine-based solvent is sufficiently mixed within the above temperature and time range, and then the mixture is allowed to stand and subjected to phase separation, and an upper layer liquid and a lower layer liquid are separated to complete the extraction.

At this time, the upper layer liquid includes an amine-based solvent and a phosphorus-based catalyst, and the lower layer liquid includes an acrylonitrile dimer.

The upper layer liquid separated with lower layer liquid may be further subjected to a reduced pressure distillation process in order to remove an amine-based solvent and recover a pure phosphorus-based catalyst.

The lower layer liquid, i.e., the acrylonitrile dimer phase, is mostly composed of 1,4-dicyanobutene (DCB), and may contain a small amount of methylene glutaronitrile (MGN). Therefore, in order to remove the MGN, the acrylonitrile dimer phase can be further purified, and subjected to a partial hydrogenation reaction to prepare adiponitrile, which is a precursor of hexamethylenediamine.

On the other hand, the reaction solvent, the proton-donating solvent and unreacted acrylonitrile distilled in step 1, and the phosphorus-based catalyst separated in step 2 may be recovered in a reaction vessel and reused. By recycling the solvent and raw materials of the reaction in this way, the production cost can be reduced and the process efficiency can be enhanced.

On the other hand, step 2 further includes a step (step 2A) of removing the low boiling point material from the reaction mixture containing the acrylonitrile dimer before the addition of the amine-based solvent. The low boiling point material removed in step 2A includesthe reaction solvent, the proton-donating solvent, and/ an unreacted acrylonitrile. When step 2A is additionally included, the reaction mixture is composed of an acrylonitrile dimer and a phosphorus-based catalyst, so that the extraction efficiency of the phosphorus-based catalyst that is subsequently proceeded can be further increased.

Hereinafter, the present disclosure will be described in more detail by way of examples in order to facilitate the understanding of the invention. However, the examples described below are for illustrative purposes only, and the content of the present disclosure is not limited thereby.

In the Examples and Comparative Examples described below, in order to confirm the extraction efficiency of the extraction solvent, which is an embodiment of the present disclosure, liquid-liquid extraction was performed with an arbitrary reaction mixture containing only the catalyst and the product.

**Experimental Example 1: Evaluation of partition coefficient of catalyst**

To investigate the partition coefficient of the phosphorus-based catalyst to the extraction solvent and acrylonitrile dimer, the following experiment was performed assuming a situation in which the hydrocarbon-based solvent, alcohol, and unreacted acrylonitrile in the reaction mixture were removed through the first purification process.

Isopropyl diphenylphosphinite (0.634 g) as the catalyst and adiponitrile (1.902 g) as the product were mixed to prepare a 25 wt.% solution of isopropyl diphenylphosphinite. To the prepared solution was added 2 ml of triethylamine as an extraction solvent, heated to 50°C, stirred for about 3 to 5 minutes, and then waited until layer separation occurred. When the layers were separated, 0.25 ml of each was extracted from an upper layer liquid (extracted) as the extraction solvent layer and a lower layer liquid (Raffinate) as the adiponitrile layer, and component analysis was performed by GC (GC 2030, Shimadzu). At this time, the detector was FID and was set to 350°C. The column used was HP-5MS and was set at 40-280°C. The temperature of the injection port was 260°C, and the mobile phase used was $N_2$.

**Comparative Experimental Example 1**

Layer separation was performed in the same manner as in Experimental Example 1, except that hexane was used as the extraction solvent.

Each of the upper layer liquid and the lower layer liquid extracted in Experimental Example 1 and Comparative Experimental Example 1 was subjected to component analysis by Gas Chromatography (GC), and the fraction of the catalyst contained in each layer was confirmed at 50°C and 101.3 kPa (760 torr). Subsequently, the partition coefficient was calculated in accordance with Equation 1 below and shown in Table 1 below.

[Equation 1]

$$K_{AM/ADN} = C_{AM}/C_{ADN}$$

in Equation 1,
$C_{AM}$ is the mass fraction of the phosphorus-based catalyst in the amine-based solvent, and
$C_{ADN}$ is the mass fraction of phosphorus-based catalyst in adiponitrile.

[Table 1]

| | Extraction solvent | Catalyst mass fraction of upper layer liquid (extracted) ($C_{AM}$) | Catalyst mass fraction of lower layer liquid (Raffinate) ($C_{ADN}$) | Partition coefficient |
|---|---|---|---|---|
| Experimental Example 1 | triethylami ne | 0.2341 | 0.0804 | 2.9117 |
| Comparative Experimental Example 1 | hexane | 0.2033 | 0.0972 | 2.0916 |

As shown in Table 1, the catalyst partition coefficient of Experimental Example 1 using triethylamine was about 1.5 times larger compared to that of Comparative Experimental Example 1 in which commonly used hexane was used as an extraction solvent. That is, it can be confirmed that the phosphorus-based catalyst was better dissolved in triethylamine than in hexane, so that when triethylamine was used as an extraction solvent, the extraction efficiency of the phosphorus-based catalyst was higher.

**Example 1**

220 ml of toluene, 66 ml of acrylonitrile, and 22 ml of isopropyl alcohol were put into a 1 L reactor, and ethyl diphenylphosphinite ($Ph_2POEt$) was added in an amount of 3 mol% based on the acrylonitrile. The mixture was stirred and reacted at 60°C for 3 hours.

After completion of the reaction, the reaction mixture was distilled under a temperature of 80°C and a pressure of 6.7 kPa (50 torr), and toluene, isopropanol, and unreacted acrylonitrile were removed from the reaction mixture.

Then, 27 ml of triethylamine was added to the reaction mixture, and then stirred at 50°C for 5 minutes. After stopping the stirring and allowing to stand so that phase separation occurs, an upper layer liquid and a lower layer liquid were separated, and the components of the upper layer liquid and the lower layer liquid were analyzed using gas chromatography (GC/FID, Shimadzu, FID: 350°C, Column: HP-5MS, 40-280°C, Injection port temperature: 260 °C, mobile phase: $N_2$).

As a result of the analysis, the lower layer liquid was confirmed to contain 79.76 wt.% of 1,4-dicyanobutene (DCB), 3.74 wt.% of methylene glutaronitrile (MGN), 8.186 wt.% of ethyl diphenylphosphinite, and 8.30 wt.% of triethylamine, and the upper layer liquid was confirmed to contain 73.17 wt.% of triethylamine, 24.38 wt.% of ethyl diphenylphosphinite, 2.331 wt.% of 1,4-dicyanobutene (DCB), and 0.109 wt.% of methylene glutaronitrile (MGN).

**Comparative Example 1**

220 ml of toluene, 66 ml of acrylonitrile, and 22 ml of isopropyl alcohol were put into a 1 L reactor, and ethyl diphenylphosphinite ($Ph_2POEt$) was added in an amount of 3 mol% based on the acrylonitrile. The mixture was stirred and reacted at 60°C for 3 hours.

After completion of the reaction, the reaction mixture was distilled under a temperature of 80°C and a pressure of 6.7 kPa (50 torr), and toluene, isopropanol, and unreacted acrylonitrile were removed from the reaction mixture.

Then, 27 ml of n-hexane was added to the reaction mixture, and then stirred at 50°C for 5 minutes. After stopping the stirring and allowing to stand so that phase separation occurs, an upper layer liquid and a lower layer liquid were separated, and the components of the upper layer liquid and the lower layer liquid were analyzed using gas chromatography (GC/FID, Shimadzu, FID: 350°C, Column: HP-5MS, 40-280°C, Injection port temperature: 260 °C, mobile phase: $N_2$).

As a result of the analysis, the lower layer liquid was confirmed to contain 83.9 wt.% of 1,4-dicyanobutene(DCB), 3.8 wt.% of methylene glutaronitrile(MGN), 9.73 wt.% of ethyl diphenylphosphinite, and 2.48 wt.% of n-hexane, and the upper layer liquid was confirmed to contain 78.71 wt.% of n-hexane, 20.39 wt.% of ethyl diphenylphosphinite, 0.8528 wt.% of 1,4-dicyanobutene (DCB), and 0.039 wt.% of methylene glutaronitrile (MGN).

According to Example 1 and Comparative Example 1, it was confirmed that the catalyst partition coefficient of Example 1 was about 2.98, the catalyst partition coefficient of Comparative Example 1 was about 2.09, and when an amine-based solvent was used as an extraction solvent, the catalyst was better separated in the upper layer liquid.

**Claims**

**1.** A method for preparing acrylonitrile dimer comprising the steps of:

reacting acrylonitrile in the presence of a reaction solvent, a proton-donating solvent, and a phosphorus-based catalyst to prepare an acrylonitrile dimer (step 1);

removing the reaction solvent, the proton-donating solvent and unreacted acrylonitrile (step 2A); and

adding an amine-based solvent to a reaction mixture containing the acrylonitrile dimer prepared in step 1 and extracting the same to separate the acrylonitrile dimer and the phosphorus-based catalyst (step 2);

wherein the reaction solvent is at least one selected from the group consisting of toluene, cyclohexane, 1,2-dichloroethane, 1,4-dioxane, chlorobenzene, and xylene,

the proton-donating solvent is at least one selected from the group consisting of isopropyl alcohol (IPA), butanol, benzyl alcohol, and cyclohexanol,

the phosphorus-based catalyst is an alkyl diphenyl phosphinite having 1 to 10 carbon atoms, and

the amine-based solvent is at least one selected from the group consisting of triethylamine and trioctylamine.

2. The method of claim 1, wherein:
the reaction solvent is toluene.

3. The method of claim 1, wherein:
the proton-donating solvent is isopropyl alcohol (IPA).

4. The method of claim 1, wherein:
the phosphorus-based catalyst is isopropyl diphenylphosphinite, or ethyl diphenylphosphinite.

5. The method of claim 1, wherein:

a partition coefficient ($K_{AM/ADN}$) of the amine-based solvent-adiponitrile of the phosphorus-based catalyst represented by Equation 1 below at 50°C and 101.3 kPa (760 torr) is 2.5 or more.

$$[Equation\ 1]$$

$$K_{AM/ADN} = C_{AM}/C_{ADN}$$

in Equation 1,
$C_{AM}$ is the mass fraction of the phosphorus-based catalyst in the amine-based solvent, and
$C_{ADN}$ is the mass fraction of phosphorus-based catalyst in adiponitrile.

6. The method of claim 1, wherein:
the amine-based solvent is triethylamine or trioctylamine.

7. The method of claim 1, wherein:
the step 2 is performed at 30°C or more and 80°C or less.

8. The method of claim 1, wherein:
the step 2 is performed for 1 minute or more and 30 minutes or less.

**Patentansprüche**

1. Verfahren zur Herstellung von Acrylnitrildimer, umfassend die Schritte:

Umsetzen von Acrylnitril in Gegenwart eines Reaktionslösungsmittels, eines protonenabgebenden Lösungsmittels und eines Katalysators auf Phosphorbasis, um ein Acrylnitrildimer herzustellen (Schritt 1);

Entfernen des Reaktionslösungsmittels, des protonenabgebenden Lösungsmittels und von nicht umgesetztem Acrylnitril (Schritt 2A); und

Zugeben eines Lösungsmittels auf Aminbasis zu einer Reaktionsmischung, die das in Schritt 1 hergestellte Acrylnitrildimer enthält, und Extrahieren desselben, um das Acrylnitrildimer und den Katalysator auf Phosphorbasis zu trennen (Schritt 2);

wobei das Reaktionslösungsmittel mindestens eines ist, das aus der Gruppe ausgewählt ist, die aus Toluol, Cyclohexan, 1,2-Dichlorethan, 1,4-Dioxan, Chlorbenzol und Xylol besteht,

das protonenabgebende Lösungsmittel mindestens eines ist, das aus der Gruppe ausgewählt ist, die aus Isopropylalkohol (IPA), Butanol, Benzylalkohol und Cyclohexanol besteht,
der Katalysator auf Phosphorbasis ein Alkyldiphenylphosphinit mit 1 bis 10 Kohlenstoffatomen ist, und
das Lösungsmittel auf Aminbasis mindestens eines ist, das aus der Gruppe ausgewählt ist, die aus Triethylamin
und Trioctylamin besteht.

**2.** Verfahren nach Anspruch 1, wobei:
das Reaktionslösungsmittel Toluol ist.

**3.** Verfahren nach Anspruch 1, wobei:
das protonenabgebende Lösungsmittel Isopropylalkohol (IPA) ist.

**4.** Verfahren nach Anspruch 1, wobei:
der Katalysator auf Phosphorbasis Isopropyldiphenylphosphinit oder Ethyldiphenylphosphinit ist.

**5.** Verfahren nach Anspruch 1, wobei:

ein Verteilungskoeffizient ($K_{AM/ADN}$) des Lösungsmittels auf Aminbasis-Adiponitrils des Katalysators auf Phosphorbasis, der durch die nachstehende Gleichung 1 dargestellt wird, bei 50 °C und 101,3 kPa (760 Torr) 2,5 oder mehr beträgt.

[Gleichung 1]

$$K_{AM/ADN} = C_{AM}/C_{ADN}$$

wobei in Gleichung 1
$C_{AM}$ der Massenanteil des Katalysators auf Phosphorbasis in dem Lösungsmittel auf Aminbasis ist und
$C_{ADN}$ der Massenanteil des Katalysators auf Phosphorbasis in Adiponitril ist.

**6.** Verfahren nach Anspruch 1, wobei:
das Lösungsmittel auf Aminbasis Triethylamin oder Trioctylamin ist.

**7.** Verfahren nach Anspruch 1, wobei:
der Schritt 2 bei 30 °C oder mehr und 80 °C oder weniger durchgeführt wird.

**8.** Verfahren nach Anspruch 1, wobei:
der Schritt 2 für 1 Minute oder mehr und 30 Minuten oder weniger durchgeführt wird.

**Revendications**

**1.** Procédé de préparation d'un dimère d'acrylonitrile comprenant les étapes consistant à :

faire réagir l'acrylonitrile en présence d'un solvant de réaction, d'un solvant donneur de protons et d'un catalyseur à base de phosphore pour préparer un dimère d'acrylonitrile (étape 1) ;
retirer le solvant de réaction, le solvant donneur de protons et l'acrylonitrile non réagi (étape 2A) ; et
ajouter un solvant à base d'amine à un mélange de réaction contenant le dimère d'acrylonitrile préparé à l'étape 1 et extraire celui-ci pour séparer le dimère d'acrylonitrile et le catalyseur à base de phosphore (étape 2) ;
dans lequel le solvant de réaction est un ou plusieurs éléments sélectionnés dans le groupe constitué de toluène, cyclohexane, 1,2-dichloroéthane, 1,4-dioxane, chlorobenzène et xylène,
le solvant donneur de protons est un ou plusieurs éléments sélectionnés dans le groupe constitué d'alcool isopropylique (IPA), butanol, alcool benzylique et cyclohexanol,
le catalyseur à base de phosphore est une phosphinite d'alkyldiphényle ayant 1 à 10 atomes de carbone, et
le solvant à base d'amine est un ou plusieurs éléments sélectionnés dans le groupe constitué de triéthylamine et trioctylamine.

**2.** Procédé selon la revendication 1, dans lequel :
le solvant de réaction est du toluène.

**3.** Procédé selon la revendication 1, dans lequel :
le solvant donneur de protons est de l'alcool isopropylique (IPA).

**4.** Procédé selon la revendication 1, dans lequel :
le catalyseur à base de phosphore est de la diphénylphosphinite isopropylique ou de la diphénylphosphinite éthylique.

**5.** Procédé selon la revendication 1, dans lequel :

un coefficient de partage ($K_{AM/ADN}$) de l'adiponitrile-solvant à base d'amine du catalyseur à base de phosphore représenté par l'Équation 1 ci-dessous à 50°C et 101,3 kPa (760 torr) est égal ou supérieur à 2,5.

## [Équation 1]

$$K_{AM/ADN} = C_{AM}/C_{ADN}$$

dans l'Équation 1,
$C_{AM}$ est la fraction massique du catalyseur à base de phosphore dans le solvant à base d'amine, et
$C_{ADN}$ est la fraction massique de catalyseur à base de phosphore dans l'adiponitrile.

**6.** Procédé selon la revendication 1, dans lequel :
le solvant à base d'amine est de la triéthylamine ou de la trioctylamine.

**7.** Procédé selon la revendication 1, dans lequel :
l'étape 2 est effectuée à 30°C ou plus et 80°C ou moins.

**8.** Procédé selon la revendication 1, dans lequel :
l'étape 2 est effectuée pendant 1 minute ou plus et 30 minutes ou moins.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4639539 A
- WO 9310082 A1
- WO 2013095853 A1